(19) Europäisches Patentamt
European Patent Office
Office européen des brevets

(11) **EP 4 566 708 A1**

(12) **EUROPEAN PATENT APPLICATION**
published in accordance with Art. 153(4) EPC

(43) Date of publication:
**11.06.2025 Bulletin 2025/24**

(21) Application number: **23850137.3**

(22) Date of filing: **03.08.2023**

(51) International Patent Classification (IPC):
*B01J 23/80* (2006.01)       *B01J 35/61* (2024.01)
*B01J 37/03* (2006.01)       *B01J 37/08* (2006.01)
*B01J 37/18* (2006.01)       *C01G 25/00* (2006.01)
*C07B 61/00* (2006.01)       *C07C 29/153* (2006.01)
*C07C 31/04* (2006.01)

(52) Cooperative Patent Classification (CPC):
**B01J 23/80; B01J 35/61; B01J 37/03; B01J 37/08; B01J 37/18; C01G 25/00; C07B 61/00; C07C 29/153; C07C 31/04; Y02P 20/52**

(86) International application number:
**PCT/JP2023/028367**

(87) International publication number:
**WO 2024/029584 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.08.2022 JP 2022125078**

(71) Applicants:
• **Mitsui Mining & Smelting Co., Ltd.**
  **Shinagawa-ku**
  **Tokyo 141-8584 (JP)**
• **Honda Motor Co., Ltd.**
  **Minato-ku**
  **Tokyo 107-8556 (JP)**

(72) Inventors:
• **KURAMOCHI, Satoshi**
  **Ageo-shi**
  **Saitama 362-0021 (JP)**

• **KOMANOYA, Tasuku**
  **Ageo-shi**
  **Saitama 362-0021 (JP)**
• **OSAKO, Takao**
  **Ageo-shi**
  **Saitama 362-0021 (JP)**
• **YAMAMOTO, Osami**
  **Wako-shi**
  **Saitama 351-0193 (JP)**
• **SUMI, Hideaki**
  **Wako-shi**
  **Saitama 351-0193 (JP)**
• **KAIDA, Chiharu**
  **Wako-shi**
  **Saitama 351-0193 (JP)**
• **MASUDA, Shohei**
  **Wako-shi**
  **Saitama 351-0193 (JP)**

(74) Representative: **Novagraaf Technologies**
  **Bâtiment O2**
  **2, rue Sarah Bernhardt**
  **CS90017**
  **92665 Asnières-sur-Seine Cedex (FR)**

(54) **COMPOSITE OXIDE, CATALYST FOR METHANOL PRODUCTION, AND COMPOSITE OXIDE PRODUCTION METHOD**

(57)    An object of the present invention is to provide a complex oxide having an improved $CO_2$ methanation catalytic performance, a catalyst for methanol production containing the complex oxide, and a complex oxide production method that is suitable for producing the complex oxide, and the present invention provides a complex oxide containing Cu, Zn and Zr, wherein the complex oxide has a specific surface area of 50 $m^2$/g or more and a Cu dispersity of 6.5% or more, a catalyst for methanol production containing the complex oxide, and a complex oxide production method that is suitable for producing the complex oxide.

EP 4 566 708 A1

## Description

### FIELD OF THE INVENTION

The present invention relates to a complex oxide, a catalyst for methanol production and a method for producing a complex oxide.

### BACKGROUND ART

A reduction in greenhouse gases such as carbon dioxide is increasingly being attempted worldwide recently. As a solution for reducing carbon dioxide, a catalyst for promoting the methanation reaction of $CO_2$ ($CO_2 + 3H_2 \rightarrow CH_3OH + H_2O$) has attracted attention, and various $CO_2$ methanation catalysts have been developed.

For example, Patent Literature 1 discloses a $CO_2$ methanation catalyst containing copper oxide, zinc oxide and zirconium oxide, and further containing at least one element selected from aluminum, chromium and palladium.

### CITATION LIST

Patent Literature

Patent Literature 1: Japanese Patent Laid-Open No. 7-39755

### SUMMARY OF INVENTION

TECHNICAL PROBLEM

Unfortunately, the $CO_2$ methanation catalyst disclosed in Patent Literature 1 has an insufficient $CO_2$ methanation catalytic performance.

Accordingly, an object of the present invention is to provide a complex oxide having an improved $CO_2$ methanation catalytic performance, a catalyst for methanol production containing the complex oxide, and a complex oxide production method that is suitable for producing the complex oxide.

SOLUTION TO PROBLEM

The present invention provides the following complex oxide, catalyst for methanol production, complex oxide production method and methanol production method.

[1] A complex oxide containing Cu, Zn and Zr, wherein the complex oxide has a specific surface area of 50 $m^2$/g or more and a Cu dispersity of 6.5% or more.

[2] The complex oxide according to [1], wherein Cu, Zn and Zr contents in the complex oxide are 40% by mole or more and 90% by mole or less, 5% by mole or more and 40% by mole or less, and 1% by mole or more and 15% by mole or less, respectively, based on a total content of all metal elements in the complex oxide.

[3] The complex oxide according to [1] or [2], further containing one or two metal elements selected from Mg and Al.

[4] The complex oxide according to any one of [1] to [3], wherein the complex oxide has a CuO crystallite size of 2.0 nm or more and 25.0 nm or less.

[5] A catalyst for methanol production, including the complex oxide according to any one of [1] to [4].

[6] A method for producing a complex oxide, the method including the following steps of:

(a) adding an acidic aqueous solution containing two or more metal ions to an alkaline aqueous solution having a pH of 7.5 or more and 11 or less to form a precipitate containing two or more metal elements; and
(b) calcining the precipitate to produce a complex oxide containing the two or more metal elements.

[7] The production method according to [6], wherein, in step (a), the alkaline aqueous solution after the addition of the acidic aqueous solution has a pH of 5 or more and 9 or less.

[8] The production method according to [6] or [7], wherein the acidic aqueous solution used in step (a) contains Cu ion, Zn ion and Zr ion, and the complex oxide produced in step (b) contains Cu, Zn and Zr.

[9] The production method according to any one of [6] to [8], wherein the complex oxide produced in step (b) further contains one or two metal elements selected from Mg and Al.

[10] The production method according to any one of [6] to [9], wherein the complex oxide produced in step (b) is the complex oxide according to any one of [1] to [4].

[11] A method for producing methanol, the method including a step of contacting $CO_2$ gas with $H_2$ gas under conditions of 100°C or more and 400°C or less and 0.1 MPa or more and 30 MPa or less in the presence of the complex oxide according to any one of [1] to [4] subjected to a reduction treatment.

ADVANTAGEOUS EFFECTS OF INVENTION

The present invention provides a complex oxide having an improved $CO_2$ methanation catalytic performance, a catalyst for methanol production containing the complex oxide, and a complex oxide production method that is suitable for producing the complex oxide.

## DETAILED DESCRIPTION OF THE INVENTION

<<Complex oxide>>

Hereinafter, a complex oxide of the present invention will be described. The "complex oxide" means an oxide containing two or more metal elements. For example, not only a complex of two or more metal oxides but also a solid solution containing two or more metal oxides are included in the "complex oxide".

<Form of complex oxide>

The complex oxide of the present invention is in the form of, for example, powder. The complex oxide of the present invention may be a molded body formed into a desired shape such as a pellet or a layer.

<Physical properties of complex oxide>

The complex oxide of the present invention preferably has one or more of the below-mentioned specific surface area, Cu dispersity and CuO crystallite size, more preferably has the below-mentioned specific surface area and Cu dispersity, and still more preferably has the below-mentioned specific surface area, Cu dispersity and CuO crystallite size.
The specific surface area of the complex oxide of the present invention is preferably 50 $m^2$/g or more, more preferably 90 $m^2$/g or more, and still more preferably 95 $m^2$/g or more, from the viewpoint of improving the diffusivity of raw material gases for methanol production ($CO_2$ and $H_2$), thereby improving the $CO_2$ methanation catalytic performance (for example, $CO_2$ conversion rate and methanol yield) of the complex oxide of the present invention. The upper limit is not limited. The specific surface area of the complex oxide of the present invention may be, for example, 130 $m^2$/g or less, 125 $m^2$/g or less, or 120 $m^2$/g or less. Each of these upper limits may be combined with any of the lower limits described above. The specific surface area can be measured, for example, in accordance with the method described in Examples.
The Cu dispersity of the complex oxide of the present invention is preferably 6.5% or more, more preferably 7.0% or more, and still more preferably 8.0% or more, from the viewpoint of improving the contact of Cu with raw material gases for methanol production ($CO_2$ and $H_2$), thereby improving the $CO_2$ methanation catalytic performance (for example, $CO_2$ conversion rate and methanol yield) of the complex oxide of the present invention. The upper limit is not limited. The Cu dispersity of the complex oxide of the present invention may be, for example, 30% or less, 20% or less, or 15% or less. Each of these upper limits may be combined with any of the lower limits described above. The Cu dispersity can be measured, for example, in accordance with the method described in Examples.
The CuO crystallite size of the complex oxide of the present invention contributes to the Cu dispersity of the complex oxide of the present invention. The CuO crystallite size of the complex oxide of the present invention is preferably 2.0 nm or more and 25.0 nm or less, more preferably 3.0 nm or more and 20.0 nm or less, and still more preferably 4.0 nm or more and 10.0 nm or less, from the viewpoint of achieving the above-mentioned Cu dispersity of the complex oxide of the present invention, thereby improving the $CO_2$ methanation catalytic performance (for example, $CO_2$ conversion rate and methanol yield) of the complex oxide of the present invention. The CuO crystallite size can be measured, for example, in accordance with the method described in Examples.

<Composition of complex oxide>

The complex oxide of the present invention contains Cu, Zn and Zr. The complex oxide of the present invention contains O in addition to Cu, Zn and Zr.

The Cu content (molar amount) of the complex oxide of the present invention is preferably 40% by mole or more and 90% by mole or less, more preferably 45% by mole or more and 80% by mole or less, and still more preferably 55% by mole or more and 75% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

In the complex oxide of the present invention, Cu may form a solid solution phase(s) (for example, a solid solution phase of CuO, ZnO and $ZrO_2$, a solid solution phase of CuO and ZnO, a solid solution phase of CuO and $ZrO_2$, or the like) with Zn, Zr and O, or form a crystalline or amorphous single phase (CuO phase) with O, or form both the solid solution phase(s) and the single phase.

The Zn content (molar amount) of the complex oxide of the present invention is preferably 5% by mole or more and 40% by mole or less, more preferably 10% by mole or more and 35% by mole or less, and still more preferably 20% by mole or more and 30% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

Cu and Zn function as adsorption sites for raw material gases for methanol production ($CO_2$ and $H_2$), and contribute to the stabilization and activation of the reactive intermediate. The interaction between Cu and Zn contributes to the Cu dispersity. The molar ratio of Cu to Zn is preferably 1.0 or more and 5.0 or less, more preferably 2.0 or more and 4.0 or less, and still more preferably 2.5 or more and 3.0 or less, from the viewpoint of increasing the interaction between Cu and Zn.

In the complex oxide of the present invention, Zn may form a solid solution phase(s) (for example, a solid solution phase of ZnO, CuO and $ZrO_2$, a solid solution phase of ZnO and CuO, a solid solution phase of ZnO and $ZrO_2$, or the like) with Cu, Zr and O, or form a crystalline or amorphous single phase (ZnO phase) with O, or form both the solid solution phase(s) and the single phase.

The Zr content (molar amount) of the complex oxide of the present invention is preferably 1% by mole or more and 15% by mole or less, more preferably 2% by mole or more and 13% by mole or less, and still more preferably 4% by mole or more and 12% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

In the complex oxide of the present invention, Zr may form a solid solution phase(s) (for example, a solid solution phase of $ZrO_2$, CuO and ZnO, a solid solution phase of $ZrO_2$ and CuO, a solid solution phase of $ZrO_2$ and ZnO, or the like) with Cu, Zn and O, or form a crystalline or amorphous single phase ($ZrO_2$ phase) with O, or form both the solid solution phase(s) and the single phase.

The complex oxide of the present invention may contain one or more other metal elements in addition to Cu, Zn and Zr.

The one or more other metal elements are preferably one or two metal elements selected from Mg and Al, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

In the case where the complex oxide of the present invention contains the one or more other metal elements, the total content (total molar amount) of Cu, Zn and Zr in the complex oxide of the present invention is preferably 70% by mole or more and 99% by mole or less, more preferably 75% by mole or more and 99% by mole or less, and still more preferably 80% by mole or more and 99% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

In the case where the complex oxide of the present invention contains the one or more other metal elements, the content (molar amount) of the one or more other metal elements in the complex oxide of the present invention is preferably 1% by mole or more and 30% by mole or less, more preferably 1% by mole or more and 25% by mole or less, and still more preferably 1% by mole or more and 20% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic

performance (for example, $CO_2$ conversion rate and methanol yield) of the complex oxide of the present invention. The expression "the content (molar amount) of the one or more other metal elements" means, in the case where the complex oxide of the present invention contains one other metal element, the content (molar amount) of the one metal element, and, in the case where the complex oxide of the present invention contains two or more other metal elements, the total content (total molar amount) of the two or more metal elements.

In the complex oxide of the present invention, the other metal element may form a solid solution phase(s) (for example, a solid solution phase of an oxide of the other metal element, CuO, ZnO and $ZrO_2$, a solid solution phase of an oxide of the other metal element, CuO and ZnO, a solid solution phase of an oxide of the other metal element, CuO and $ZrO_2$, a solid solution phase of an oxide of the other metal element, ZnO and $ZrO_2$, a solid solution phase of an oxide of the other metal element and CuO, a solid solution phase of an oxide of the other metal element and ZnO, a solid solution phase of an oxide of the other metal element and $ZrO_2$, or the like) with Cu, Zn, Zr and O, or form a crystalline or amorphous single phase (an oxide phase of the other metal element) with O, or form both the solid solution phase(s) and the single phase. An oxide of Mg is MgO, and an oxide of Al is $Al_2O_3$.

In the case where the complex oxide of the present invention contains Mg, the Mg content (molar amount) of the complex oxide of the present invention is preferably 0.1% by mole or more and 10% by mole or less, more preferably 0.5% by mole or more and 7% by mole or less, and still more preferably 1% by mole or more and 5% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

In the case where the complex oxide of the present invention contains Al, the Al content (molar amount) of the complex oxide of the present invention is preferably 0.1% by mole or more and 30% by mole or less, more preferably 1% by mole or more and 25% by mole or less, and still more preferably 5% by mole or more and 20% by mole or less, based on the total content (total molar amount) of all metal elements in the complex oxide of the present invention, from the viewpoint of achieving one or more of the above-mentioned specific surface area, Cu dispersity and CuO crystallite size, thereby improving the $CO_2$ methanation catalytic performance (for example, $CO_2$ conversion rate and methanol yield) of the complex oxide of the present invention.

The content of each metal element in the complex oxide of the present invention can be measured by an ordinary method such as inductively coupled plasma optical emission spectroscopy (ICP-AES or ICP-OES).

<<Catalyst for methanol production>>

Hereinafter, a catalyst for methanol production of the present invention will be described.

The catalyst for methanol production of the present invention contains the complex oxide of the present invention. Accordingly, the catalyst for methanol production of the present invention can exhibit high $CO_2$ methanation catalytic performance (for example, $CO_2$ conversion rate and methanol yield).

The catalyst for methanol production of the present invention catalyzes the reaction that produces methanol from raw material gases for methanol production ($CO_2$ and $H_2$), namely the methanol synthesizing reaction represented by the following formula:

$$CO_2 + 3H_2 \rightarrow CH_3OH + H_2O$$

Cu and Zn function as adsorption sites for raw material gases for methanol production ($CO_2$ and $H_2$), and contribute to the stabilization and activation of the reactive intermediate. In addition, the interaction between Cu and Zn contributes to the Cu dispersity. The interaction between Cu and Zn is important in the catalytic action of the complex oxide of the present invention.

Zr increases the interaction between Cu and Zn, and functions as an adsorption site for $CO_2$. In addition, the interaction between Cu and Zr contributes to the stabilization of adsorption sites for raw material gases for methanol production ($CO_2$ and $H_2$).

Mg and Al increase the interaction between Cu and Zn.

<<Method for producing methanol>>

Hereinafter, a methanol production method of the present invention will be described.

The methanol production method of the present invention includes a step of contacting $CO_2$ gas with $H_2$ gas in the presence of the complex oxide of the present invention subjected to a reduction treatment.

In order to reduce an oxide of Cu (namely CuO) contained in the complex oxide of the present invention to thereby form Cu having a valence lower than before the reduction, the reduction treatment against the complex oxide of the present invention is conducted before use of the complex oxide of the present invention for the methanol synthesizing reaction. The reduction treatment can be conducted by, for example, heat-treating the complex oxide of the present invention in a hydrogen atmosphere at a temperature of 100°C or more and 300°C or less for 0.5 hours or more and 4 hours or less. The reduction treatment against the complex oxide of the present invention may be conducted twice or more. A specific example of the conditions for each reduction treatment is as mentioned above.

By contacting $CO_2$ gas with $H_2$ gas in the presence of the complex oxide of the present invention subjected to a reduction treatment, the methanol synthesizing reaction can progress, and thus methanol can be produced. The methanol synthesizing reaction is as mentioned above.

The conditions for the methanol synthesizing reaction are not limited as long as the methanol synthesizing reaction can progress. The temperature is usually 100°C or more and 400°C or less, and preferably 150°C or more and 350°C or less. The pressure is usually 0.1 MPa or more and 30 MPa or less, and preferably 2 MPa or more and 20 MPa or less.

<<Method for producing complex oxide>>

The production method of the present invention includes the following steps of:

(a) adding an acidic aqueous solution containing two or more metal ions to an alkaline aqueous solution having a pH of 7.5 or more and 11 or less to form a precipitate containing two or more metal elements; and

(b) calcining the precipitate formed in step (a) to produce a complex oxide containing the two or more metal elements.

The production method of the present invention is suitable for production of a complex oxide containing Cu, Zn and Zr, especially for production of the complex oxide of the present invention. The method is also usable for producing other complex oxides.

<Step (a)>

The step (a) is a step of adding an acidic aqueous solution containing two or more metal ions (hereinafter referred to as an "acidic aqueous solution (B)") to an alkaline aqueous solution having a pH of 7.5 or more and 11 or less (hereinafter referred to as an "alkaline aqueous solution (A)") to form a precipitate containing two or more metal elements. The alkaline aqueous solution (A) after the addition of the acidic aqueous solution (B) (namely a mixed solution of the alkaline aqueous solution (A) and the acidic aqueous solution (B)) may be referred to as a "reaction solution".

The alkaline aqueous solution (A) can be prepared by, for example, dissolving a base in water, and then adjusting the pH of the obtained aqueous solution as needed.

The base for preparing the alkaline aqueous solution (A) is not limited as long as it is dissolved in water or reacts with water to generate hydroxide ion, and can be selected from, for example, a carbonate, a bicarbonate, a hydroxide salt, an ammonium salt, and the like. The base is preferably selected from a carbonate and a bicarbonate, from the viewpoint of the convenience of synthetic operation and improvement in specific surface area of the complex oxide to be produced. One base may be used alone, or two or more bases may be used in combination.

The carbonate can be selected from, for example, an alkali metal salt such as sodium carbonate or potassium carbonate, an ammonium salt such as ammonium carbonate or ammonium bicarbonate, and the like. The carbonate is preferably selected from sodium carbonate and potassium carbonate, and is more preferably sodium carbonate, from the viewpoint of the convenience of synthetic operation. One carbonate may be used alone, or two or more carbonates may be used in combination.

The bicarbonate can be selected from, for example, an alkali metal salt such as sodium bicarbonate or potassium bicarbonate, ammonium bicarbonate, and the like. The bicarbonate is preferably selected from an alkali metal salt such as sodium bicarbonate and potassium bicarbonate, and is more preferably sodium bicarbonate, from the viewpoint of reducing the pH variation of the reaction solution. One bicarbonate may be used alone, or two or more bicarbonates may be used in combination.

The pH of the alkaline aqueous solution (A) is not limited as long as it is 7.5 or more and 11 or less, and is preferably 7.5 or more and 10 or less, more preferably 7.5 or more and 9 or less, and still more preferably 7.5 or more and 8.5 or less, from the viewpoint of adjusting the pH of the reaction solution to the neutral point rapidly.

The aqueous solution obtained by dissolving the base in water can be used as the alkaline aqueous solution (A) as it is if the pH is in a predetermined range. If the pH is out of the range, the aqueous solution can be used as the alkaline aqueous solution (A) after adjusting the pH to the predetermined range with an acid. The acid for adjusting the pH can be selected from, for example, nitric acid, hydrochloric acid, hydrobromic acid, sulfuric acid, phosphoric acid, and the like. The acid is preferably nitric acid, from the viewpoint of conducting efficiently the below-mentioned washing of the precipitate.

The alkaline aqueous solution (A) may contain one or more solvents other than water. The one or more solvents other than water can be selected from, for example, organic solvents such as an alcohol. The total content of the one or more solvents other than water is usually 10% by volume or less, and preferably 5% by volume or less, based on the volume of the alkaline aqueous solution (A). The lower limit is zero.

The metal element derived from the alkaline aqueous solution (A) (for example, an alkali metal element such as Na or K) is not a metal element of interest to be incorporated into the complex oxide. Accordingly, it is preferable to remove the metal element derived from the alkaline aqueous solution (A) (for example, an alkali metal element such as Na or K) from the precipitate. The removal thereof can be conducted by washing the precipitate as described below.

The two or more metal ions contained in the acidic aqueous solution (B) are metal ions corresponding to the metal elements of interest to be incorporated into the complex oxide. The two or more metal ions contained in the acidic aqueous solution (B) are not limited as long as the metal ions can each react with one or more metal ions selected from hydroxide ion, carbonate ion and bicarbonate ion in the alkaline aqueous solution (A), and optionally with one or more other metal ions contained in the acidic aqueous solution (B), to form a poorly water-soluble hydroxide, a poorly water-soluble carbonate, a poorly water-soluble bicarbonate or a poorly water-soluble carbonate hydroxide salt. The two or more metal ions contained in the acidic aqueous solution (B) can be selected from, for example, Cu, Zn, Zr, Mg, Al and the like. For example, one metal ion $M1^{2+}$ can react with one or more metal ions selected from hydroxide ion, carbonate ion and bicarbonate ion in the alkaline aqueous solution (A), and optionally with another metal ion $M2^{2+}$ contained in the acidic aqueous solution (B), to form $M1(OH)_2$, $M1CO_3$, $[(M1_xM2_{1-x})_2(OH)_2(CO_3)]$ ($0 < x \leq 1$), or the like.

In the case where the complex oxide to be produced by the production method of the present invention is a complex oxide containing Cu, Zn and Zr, especially a complex oxide of the present invention, the acidic aqueous solution (B) contains Cu ion, Zn ion and Zr ion.

In the case where the complex oxide to be produced by the production method of the present invention contains one or more other metal elements (for example, Mg, Al and the like) in addition to Cu, Zn and Zr, the acidic aqueous solution (B) can contain one or more other metal ions (for example, Mg ion, Al ion and the like) in addition to Cu ion, Zn ion and Zr ion.

The acidic aqueous solution (B) can be prepared by dissolving two or more water-soluble metal salts in water, and then adjusting the pH of the obtained aqueous solution as needed.

The water-soluble metal salts for preparing the acidic aqueous solution (B) are not limited as long as they are dissolved in water to generate metal ions of interest. Although the water-soluble metal salt may be a salt that is dissolved in water to give a neutral aqueous solution, the water-soluble metal salt is preferably a salt that is dissolved in water to give an acidic aqueous solution, from the viewpoint of convenient preparation of the acidic aqueous solution (B).

In the case where the complex oxide to be produced by the production method of the present invention contains Cu, Zn, Zr and optionally one or more other metal elements (for example, Mg, Al and the like), the acidic aqueous solution (B) can be prepared by dissolving a water-soluble Cu salt, a water-soluble Zn salt, a water-soluble Zr salt and optionally one or more other water-soluble metal salts (for example, a water-soluble Mg salt, a water-soluble Al salt and the like) in water, and then adjusting the pH of the obtained aqueous solution as needed.

The water-soluble Cu salt can be selected from, for example, copper nitrate, copper acetate, copper chloride, copper sulfate, copper carbonate and the like. Although the water-soluble Cu salt may be a salt that is dissolved in water to give a neutral aqueous solution, the water-soluble Cu salt is preferably a salt that is dissolved in water to give an acidic aqueous solution, from the viewpoint of convenient preparation of the acidic aqueous solution (B). The water-soluble Cu salt that is dissolved in water to give an acidic aqueous solution can be selected from, for example, copper nitrate, copper sulfate, copper acetate, copper chloride and the like, and is preferably selected from copper nitrate and copper acetate, from the viewpoint of convenient preparation of the acidic aqueous solution (B). One water-soluble Cu salt may be used alone, or two or more water-soluble Cu salts may be used in combination.

The water-soluble Zn salt can be selected from, for example, zinc nitrate, zinc carbonate, zinc acetate and the like. Although the water-soluble Zn salt may be a salt that is dissolved in water to give a neutral aqueous solution, the water-soluble Zn salt is preferably a salt that is dissolved in water to give an acidic aqueous solution, from the viewpoint of convenient preparation of the acidic aqueous solution (B). The water-soluble Zn salt that is dissolved in water to give an acidic aqueous solution can be selected from, for example, zinc nitrate, zinc acetate and the like, and is preferably zinc nitrate, from the viewpoint of convenient preparation of the acidic aqueous solution (B). One water-soluble Zn salt may be used alone, or two or more water-soluble Zn salts may be used in combination.

The water-soluble Zr salt can be selected from, for example, zirconium oxynitrate, zirconium carbonate, zirconium chloride and the like. Although the water-soluble Zr salt may be a salt that is dissolved in water to give a neutral aqueous solution, the water-soluble Zr salt is preferably a salt that is dissolved in water to give an acidic aqueous solution, from the viewpoint of convenient preparation of the acidic aqueous solution (B). The water-soluble Zr salt that is dissolved in water to give an acidic aqueous solution can be selected from, for example, zirconium oxynitrate, zirconium chloride and the like, and is preferably zirconium oxynitrate, from the viewpoint of convenient preparation of the acidic aqueous solution (B). One water-soluble Zr salt may be used alone, or two or more water-soluble Zr salts may be used in combination.

The other water-soluble metal salts can be selected from, for example, nitrates (for example, magnesium nitrate, aluminum nitrate and the like), carbonates (for example, magnesium carbonate, aluminum carbonate and the like) and acetates (for example, magnesium acetate, aluminum acetate and the like). Although the other water-soluble metal salts may be salts that are dissolved in water to give neutral aqueous solutions, the other water-soluble metal salts are preferably salts that are dissolved in water to give acidic aqueous solutions, from the viewpoint of convenient preparation of the acidic aqueous solution (B). The other water-soluble metal salts that are dissolved in water to give acidic aqueous solutions can be selected from, for example, nitrates (for example, magnesium nitrate, aluminum nitrate and the like) and the like, and are preferably nitrates (for example, magnesium nitrate, aluminum nitrate and the like), from the viewpoint of convenient preparation of the acidic aqueous solution (B). One other water-soluble metal salt may be used alone, or two or more other water-soluble metal salts may be used in combination.

The pH of the acidic aqueous solution (B) is not limited as long as a precipitate is formed upon mixing of the alkaline aqueous solution (A) with the acidic aqueous solution (B). The pH is preferably 5 or less, more preferably 4 or less, and still more preferably 2 or less, further preferably 1 or less, particularly preferably 0.7 or less. The lower limit is not limited. The pH of the acidic aqueous solution (B) may be, for example, 0.1 or more, 0.3 or more, 0.4 or more, or 0.5 or more. Each of these lower limits may be combined with any of the above-mentioned upper limits.

The aqueous solution obtained by dissolving the two or more water-soluble metal salts in water can be used as the acidic aqueous solution (B) as it is if the pH is in a predetermined range. If the pH is out of the range, the aqueous solution can be used as the acidic aqueous solution (B) after adjusting the pH to the predetermined range with an acid. The acid for adjusting the pH can be selected from, for example, nitric acid, sulfuric acid, hydrochloric acid, phosphoric acid and the like. The acid is preferably nitric acid, from the viewpoint of efficiently conducting the below-mentioned washing of the precipitate.

The acidic aqueous solution (B) may contain one or more solvents other than water. The one or more solvents other than water can be selected from, for example, organic solvents such as an alcohol. The total content of the one or more solvents other than water is usually 30% by volume or less, and preferably 5% by volume or less, based on the volume of the acidic aqueous solution (B). The lower limit is zero.

Upon the addition of the alkaline aqueous solution (B) to the acidic aqueous solution (A), the metal ions contained in the acidic aqueous solution (B) react with one or more ions selected from hydroxide ion, carbonate ion and bicarbonate ion contained in the alkaline aqueous solution (A), and optionally with one or more other metal ions contained in the acidic aqueous solution (B), to form a poorly water-soluble hydroxide, a poorly water-soluble carbonate, a poorly water-soluble bicarbonate or a poorly water-soluble carbonate hydroxide salt as the precipitate. That is, the precipitate containing the two or more metal elements is formed. The precipitate contains particles of one or more selected from the poorly water-soluble hydroxide, the poorly water-soluble carbonate, the poorly water-soluble bicarbonate and the poorly water-soluble carbonate hydroxide salt. The particles of the two or more selected from the poorly water-soluble hydroxide, the poorly water-soluble carbonate, the poorly water-soluble bicarbonate and the poorly water-soluble carbonate hydroxide salt may form complex particles. The two or more metal elements contained in the precipitate correspond to the two or more metal ions contained in the acidic aqueous solution (B).

In the case where the acidic aqueous solution (B) contains Cu ion, Zn ion, Zr ion and optionally one or more other metal ions, upon the addition of the acidic aqueous solution (B) to the alkaline aqueous solution (A), the metal ions contained in the acidic aqueous solution (B) react with one or more metal ions selected from hydroxide ion, carbonate ion and bicarbonate ion contained in the alkaline aqueous solution (A), and optionally with one or more other metal ions contained in the acidic aqueous solution (B), to form a poorly water-soluble hydroxide (copper hydroxide, zinc hydroxide, zirconium hydroxide or hydroxide(s) of the one or more other metal elements), a poorly water-soluble carbonate (copper carbonate, zinc carbonate, zirconium carbonate or carbonate(s) of the one or more other metal elements), a poorly water-soluble bicarbonate (copper bicarbonate, zinc bicarbonate, zirconium bicarbonate or bicarbonate(s) of the one or more other metal elements) or a poorly water-soluble carbonate hydroxide salt (copper carbonate hydroxide, zinc carbonate hydroxide, zirconium carbonate hydroxide or carbonate hydroxide salt(s) of the one or more other metal elements) as the precipitate. That is, the precipitate containing Cu, Zn, Zr and optionally one or more other metal elements is formed. The precipitate contains particles of one or more selected from the poorly water-soluble hydroxide, the poorly water-soluble carbonate, the poorly water-soluble bicarbonate and the poorly water-soluble carbonate hydroxide of the above-mentioned metals. The particles of the two or more selected from the poorly water-soluble hydroxide, the poorly water-soluble carbonate, the poorly water-soluble bicarbonate and the poorly water-soluble carbonate hydroxide salt may form complex particles.

The pH of the alkaline aqueous solution (A) after the addition of the acidic aqueous solution (B) (namely the mixed solution of the alkaline aqueous solution (A) and the acidic aqueous solution (B)) is preferably 5 or more and 9 or less, and more preferably 7 or more and 8 or less, from the viewpoint of uniforming the structure and composition of the generated reactive intermediate. The pH of the alkaline aqueous solution (A) after the addition of the acidic aqueous solution (B) means the pH of the alkaline aqueous solution (A) immediately after the addition of the acidic aqueous solution (B) to the alkaline aqueous solution (A). The pH of the reaction solution may increase with time after the addition of the acidic aqueous solution (B). If the precipitate is aged, the pH of the reaction solution after the aging of the precipitate may increase by 1 to 2 from the pH of the reaction solution before the aging of the precipitate.

The acidic aqueous solution (B) is preferably added dropwise to the alkaline aqueous solution (A) while the alkaline aqueous solution (A) is stirred during the addition of the acidic aqueous solution (B) to the alkaline aqueous solution (A), from the viewpoint of preparing the reaction solution having a uniform pH as a whole and improving the specific surface area of the complex oxide to be produced.

The temperature of the alkaline aqueous solution (A) is preferably 10°C or more and 90°C or less, more preferably 25°C or more and 80°C or less, and still more preferably 30°C or more and 70°C or less during the addition of the acidic aqueous solution (B), from the viewpoint of promoting the formation of the reactive intermediate.

The temperature of the acidic aqueous solution (B) is preferably 10°C or more and 90°C or less, more preferably 25°C or more and 80°C or less, and still more preferably 30°C or more and 70°C or less during the addition of the acidic aqueous solution (B) to the alkaline aqueous solution (A), from the viewpoint of achieving uniform formation of the reactive intermediate.

It is preferable to age the precipitate while stirring the alkaline aqueous solution (A) after the addition of the acidic aqueous solution (B), from the viewpoint of achieving uniform formation of the reactive intermediate. The aging time is preferably 1 hour or more and 24 hours or less, more preferably 1 hour or more and 20 hours or less, and still more preferably 1 hour or more and 12 hours or less. The aging temperature is preferably 10°C or more and 90°C or less, more preferably 25°C or more and 80°C or less, and still more preferably 30°C or more and 70°C or less.

The volume of the alkaline aqueous solution (A) to be provided, the concentrations of the metal ions contained in the acidic aqueous solution (B), the volume of the acidic aqueous solution (B) to be added to the alkaline aqueous solution (A), and the like can be suitably adjusted depending on, for example, the composition of the complex oxide to be produced by the production method of the present invention, and the like.

Before the precipitate obtained in step (a) is subjected to step (b), the precipitate obtained in step (a) is preferably washed with a washing solution. Water can be used as the washing solution. The precipitate can be washed by contacting the precipitate with the washing solution (for example, by mixing the precipitate with the washing solution). By contacting the precipitate with the washing solution and then performing solid-liquid separation, the washed precipitate can be obtained in the form of a cake. The solid-liquid separation can be selected from, for example, filtration, centrifugation, decantation and the like. It is preferable to wash the precipitate until the filtrate or supernatant obtained by contacting the precipitate with the washing solution and then performing solid-liquid separation has a pH of 8.5 or less (especially 7.5 or less). In addition, it is preferable to wash the precipitate until the amount of the metal element (for example, an alkali metal element such as Na or K) derived from the alkaline aqueous solution (A) contained in the filtrate or supernatant obtained by contacting the precipitate with the washing solution and then performing solid-liquid separation is 15 ppm or less (especially 10 ppm or less).

Before the precipitate obtained in step (a) is subjected to step (b), the precipitate obtained in step (a) is preferably formed into the form of a cake, followed by drying the cake. In the case where the precipitate is washed, the washed precipitate is preferably formed into the form of a cake, followed by drying the cake. The precipitate can be dried in accordance with an ordinary method. The drying temperature and the drying time are not limited as long as the cake is fully dried. For example, the drying temperature is usually 70°C or more and 200°C or less, and preferably 80°C or more and 150°C or less, and the drying time is usually 5 hours or more and 30 hours or less, and preferably 10 hours or more and 25 hours or less. The water content can be measured for determining whether the cake is fully dried. The water content after the drying is preferably 0% by mass or more and 5% by mass or less.

The dried cake may be pulverized as needed before the dried cake is subjected to step (b). The pulverization can be performed in accordance with an ordinary method. The pulverization can be performed with, for example, a mortar, a hammer mill, a ball mill, a bead mill, a jet mill, a roller mill or the like in a dry or wet process.

<Step (b)> The step (b) is a step of calcining the precipitate formed in step (a) to produce a complex oxide containing the two or more metal elements.

The precipitate can be calcined in accordance with an ordinary method. The precipitate is usually calcined in an air atmosphere. The calcining temperature is usually 100°C or more and 700°C or less, preferably 200°C or more and 600°C or less, and more preferably 300°C or more and 500°C or less. The calcining time is usually 1 hour or more and 10 hours or less, preferably 1 hour or more and 8 hours or less, and more preferably 2 hours or more and 6 hours or less.

The calcined precipitate may be pulverized as needed. The pulverization can be performed in accordance with an ordinary method. The pulverization can be performed with, for example, a mortar, a hammer mill, a ball mill, a bead mill, a jet mill, a roller mill or the like in a dry or wet process.

By calcining the precipitate, the complex oxide containing the two or more metal elements can be obtained. The two or more metal elements contained in the complex oxide correspond to the two or more metal ions contained in the acidic aqueous solution (B).

In the case where the acidic aqueous solution (B) contains Cu ion, Zn ion, Zr ion and optionally one or more other metal ions (for example, Mg ion, Al ion and the like), a complex oxide containing Cu, Zn, Zr and optionally one or more other metal elements (for example, Mg, Al and the like), especially the complex oxide of the present invention, can be obtained.

The production method of the present invention makes it possible to produce a complex oxide having one or more of the above-mentioned specific surface area, Cu dispersity and CuO crystallite size. The complex oxide produced by the production method of the present invention can exhibit high CO2 methanation catalytic performance (for example, $CO_2$ conversion rate and methanol yield).

**EXAMPLES**

[Example 1]

(1) Precipitation forming step

In a container, 504.1 g of sodium bicarbonate was dissolved in 6 L of pure water to prepare a 1 mol/L alkaline aqueous solution (liquid temperature: 40°C and pH: 8.3). In another container, 223.1 g (0.92 mol) of copper nitrate trihydrate, 103.0 g (0.35 mol) of zinc nitrate hexahydrate, and 61.7 g (0.23 mol) of zirconium oxynitrate dihydrate were dissolved in 1.5 L of pure water to prepare an acidic aqueous solution (liquid temperature: 40°C and pH: 0.6). The alkaline aqueous solution was heated to 60°C, followed by adding the acidic aqueous solution dropwise to the alkaline aqueous solution with a tube pump at a constant rate for 40 minutes while stirring the alkaline aqueous solution. Thus, a precipitate was formed to obtain a suspension (liquid temperature: 60°C and pH: 7.1).

(2) Washing step

The precipitate was aged while the obtained suspension was stirred at 60°C for 2 hours. The suspension after the aging was air-cooled, followed by removal of the supernatant. To the suspension remaining the container was added around 10 L of pure water, and the mixture was left to stand for 1 hour, followed by removing the supernatant again. This washing operation was repeated until the pH of the supernatant was 7 or less, and the concentration of Na ion in the supernatant was equal to or less than the limit of detection.

(3) Complex oxide producing step

The suspension subjected to the washing step was suction-filtered, followed by drying the obtained cake at 100°C in a drying warehouse overnight. The dried lump was pulverized with an agate mortar. The powder after the pulverization was calcined in an air atmosphere at 400°C for 4 hours to obtain a complex oxide containing Cu, Zn and Zr.

[Example 2]

A complex oxide containing Cu, Zn, Zr and Mg was obtained by the same operation as in Example 1 except that a precipitate forming step was performed as follows.
The precipitate forming step in Example 2 was as follows.
In a container, 336.0 g of sodium bicarbonate was dissolved in 4 L of pure water to prepare a 1 mol/L alkaline aqueous solution (liquid temperature: 25°C and pH: 8.0). In another container, 144.2 g (0.60 mol) of copper nitrate trihydrate, 66.6 g (0.22 mol) of zinc nitrate hexahydrate, 39.9 g (0.15 mol) of zirconium oxynitrate dihydrate, and 7.7 g (0.03 mol) of magnesium nitrate were dissolved in 1 L of pure water to prepare an acidic aqueous solution (liquid temperature: 40°C and pH: 0.6). The alkaline aqueous solution was heated to 60°C, followed by adding the acidic aqueous solution dropwise to the alkaline aqueous solution with a tube pump at a constant rate for 25 minutes while stirring the alkaline aqueous solution. Thus, a precipitate was formed to obtain a suspension (liquid temperature: 60°C and pH: 7.2).

[Example 3]

A complex oxide containing Cu, Zn, Zr and Al was obtained by the same operation as in Example 1 except that a precipitate forming step was performed as follows.
The precipitate forming step in Example 3 was as follows.
In a container, 336.0 g of sodium bicarbonate was dissolved in 4 L of pure water to prepare a 1 mol/L alkaline aqueous solution (liquid temperature: 25°C and pH: 8.0). In another container, 195.1 g (0.81 mol) of copper nitrate trihydrate, 103.0 g (0.35 mol) of zinc nitrate hexahydrate, 46.3 g (0.17 mol) of zirconium oxynitrate dihydrate, and 64.9 g (0.17 mol) of aluminum nitrate nonahydrate were dissolved in 1.5 L of pure water to prepare an acidic aqueous solution (liquid temperature: 25°C and pH: 0.4). The alkaline aqueous solution was heated to 60°C, followed by adding the acidic aqueous solution dropwise to the alkaline aqueous solution with a tube pump at a constant rate for 25 minutes while stirring the alkaline aqueous solution. Thus, a precipitate was formed to obtain a suspension (liquid temperature: 60°C and pH: 7.2).

[Comparative Example 1]

A complex oxide containing Cu, Zn and Zr was obtained by the same operation as in Example 1 except that a precipitate forming step was performed as follows.

A precipitate forming step in Comparative Example 1 was as follows.

In a container, 208.1 g (0.86 mol) of copper nitrate trihydrate, 96.1 g (0.32 mol) of zinc nitrate hexahydrate, and 57.6 g (0.22 mol) of zirconium oxynitrate dihydrate were dissolved in 1.4 L of pure water to prepare an acidic aqueous solution (liquid temperature: 25°C and pH: 0.4). In another container, a 1.2 mol/L aqueous sodium carbonate solution was prepared. The acidic aqueous solution was heated to 60°C, followed by adding the aqueous sodium carbonate solution to the acidic aqueous solution rapidly while stirring the acidic aqueous solution vigorously. Thus, a precipitate was formed to prepare a suspension. The aqueous sodium carbonate solution was continuously added until the pH of the suspension was steady around 8.0.

[Comparative Example 2]

A complex oxide containing Cu, Zn, Zr and Mg was obtained by the same operation as in Example 1 except that a precipitate forming step was performed as follows.

A precipitate forming step in Comparative Example 2 was as follows.

In a container, 201.9 g (0.84 mol) of copper nitrate trihydrate, 93.2 g (0.31 mol) of zinc nitrate hexahydrate, 55.8 g (0.21 mol) of zirconium oxynitrate dihydrate, and 10.7 g (0.04 mol) of magnesium nitrate hexahydrate were dissolved in 1.4 L of pure water to prepare an acidic aqueous solution (liquid temperature: 25°C and pH: 0.4). In another container, a 1.2 mol/L aqueous sodium carbonate solution was prepared. The acidic aqueous solution was heated to 60°C, followed by adding the aqueous sodium carbonate solution to the acidic aqueous solution rapidly while stirring the acidic aqueous solution vigorously. Thus, a suspension was prepared thereby. The aqueous sodium carbonate solution was continuously added until the pH of the suspension was steady around 8.0.

[Comparative Example 3]

A complex oxide containing Cu, Zn, Zr and Al was obtained by the same operation as in Example 1 except that a precipitate forming step was performed as follows.

The precipitate forming step in Comparative Example 3 was as follows.

In a container, 182.1 g (0.75 mol) of copper nitrate trihydrate, 96.1 g (0.32 mol) of zinc nitrate hexahydrate, 43.2 g (0.16 mol) of zirconium oxynitrate dihydrate, and 60.6 g (0.16 mol) of aluminum nitrate nonahydrate were dissolved in 1.4 L of pure water to prepare an acidic aqueous solution (liquid temperature: 30°C and pH: 0.4). In another container, a 1.2 mol/L aqueous sodium carbonate solution was prepared. The acidic aqueous solution was heated to 60°C, followed by adding the aqueous sodium carbonate solution to the acidic aqueous solution rapidly while stirring the acidic aqueous solution vigorously. The aqueous sodium carbonate solution was continuously added until the pH of the suspension was steady around 8.0.

[Comparative Example 4]

A complex oxide containing Cu, Zn and Zr was obtained by the same operation as in Example 1 except that a precipitate forming step was performed as follows.

The precipitate forming step in Comparative Example 4 was as follows.

In a container, 268.0 g of sodium carbonate was dissolved in 2.1 L of pure water to prepare a 1.2 mol/L aqueous sodium carbonate solution (liquid temperature: 25°C and pH: 11.7). In another container, 193.3 g (0.80 mol) of copper nitrate trihydrate, 89.3 g (0.30 mol) of zinc nitrate hexahydrate, and 75.0 g (0.28 mol) of zirconium oxynitrate dihydrate were dissolved in 1.4 L of pure water to prepare an acidic aqueous solution (liquid temperature: 30°C and pH: 0.5). The aqueous sodium carbonate solution was heated to 60°C, followed by adding the acidic aqueous solution dropwise to the aqueous sodium carbonate solution with a tube pump at a constant rate for 55 minutes while stirring the aqueous sodium carbonate solution. Thus, a precipitate was formed to obtain a suspension (liquid temperature: 60°C and pH: 7.5).

[Test Example 1]

With respect to each of the complex oxides obtained in Examples 1 to 3 and Comparative Examples 1 to 4, the content of each metal element, the specific surface area, the Cu dispersity and the CuO crystallite size were measured. The results are shown in Table 1. The methods for measuring the content of each metal element, the specific surface area, the Cu dispersity and the CuO crystallite size were as follows.

<Content of each metal element>

The content (mass) of each metal element in each of the complex oxides was measured by inductively coupled plasma optical emission spectroscopy (ICP-AES or ICP-OES). The content (molar amount) of each metal element was calculated based on the measured values. The results of the calculation are shown in Table 1. In Table 1, the contents (molar amounts) of all the metal elements contained in each of some complex oxides do not total 100% for the following reason: the content of each element was rounded to the nearest whole number.

<Specific surface area>

In order to remove moisture contained in each of the complex oxides, each of the complex oxides was heat-treated in an air atmosphere at 120°C for 10 minutes. Each of the heat-treated complex oxides was measured for specific surface area. The specific surface area was measured with a commercial BET specific surface area measuring apparatus (BELSORP MR6, which was available from MicrotracBEL Corp.) by the BET one-point method. The BET one-point method was performed in accordance with "(3.5) One-point method" in "6.2 Flow process" in JIS R1626:1996 "Measuring methods for the specific surface area of fine ceramic powders by gas adsorption using the BET method". The one-point method was performed at an equilibrium relative pressure of 0.05 to 0.35 using nitrogen-helium mixed gas containing 30% by volume nitrogen, which was adsorption gas, and 70% by volume helium, which was carrier gas, as the gas for the one-point method.

<Cu dispersity>

The Cu dispersity was measured with a commercial metal dispersity measuring apparatus (BELMETAL3, which was available from MicrotracBEL Corp.) by $N_2O$ pulse adsorption. $N_2O$ pulse adsorption is a method involving pulsed introduction of $N_2O$ gas into a sample until the saturation for calculating the Cu dispersity on the sample from the total amount of the gas consumed. The results of the $N_2O$ pulse adsorption measurement were analyzed in the automatic analysis mode of the waveform analyzing software "ChemMaster" attached to the apparatus. The dispersity was calculated, supposing that the stoichiometric ratio was 2, the stoichiometric ratio indicating the number of $N_2O$ molecules to be adsorbed on one Cu metal atom. The analysis was performed in accordance with "Instruction Manual for Waveform Analyzing Software ChemMaster (Ver. 1.3) by MicrotracBEL Corp.".

The Cu dispersity as used here means the percentage of "B" to "A" wherein "B" represents the number of Cu atoms exposed to the surface of the complex oxide, and "A" represents the total number of Cu atoms in the complex oxide. The percentage is calculated in accordance with the following expression:

$$\text{Cu dispersity (\%)} = (B/A) \times 100$$

"B", the number of Cu atoms exposed to the surface of the complex oxide, is calculated from the adsorbed amount of $N_2O$ measured by the $N_2O$ pulse method based on the assumption that a Cu atom exposed to the surface of the complex oxide adsorbs $N_2O$ at a ratio of 1:2. The calculation method is as follows.

(Calculation method)

From "$V_t$" representing the amount of the $N_2O$ gas adsorbed by the amount of the sample "W" (g) at the measurement temperature, "V" representing the amount of gas adsorbed per 1 g of the complex oxide at 0°C was calculated in accordance with the following expression (1):

$$V \text{ (mL/g)} = (V_t/W) \times \{273/(273 + t)\} \qquad (1)$$

Here, assuming that the Cu content of the sample is "C" (% by mass) and the atomic weight of Cu is "M", "R" representing the mole number of Cu per 1 g of the sample is calculated in accordance with the following expression (2):

$$R \ (mol/g) = (C/100) \times (1/M) \qquad (2)$$

"K" representing the mole number of the amount of gas adsorbed per 1 g of the sample is calculated in accordance with the following expression (3):

$$K \ (mol/g) = V/(22.4 \times 10^{-3} \times 10^{6}) \qquad (3)$$

From the above, "D" representing the Cu dispersity is calculated using "S" representing the stoichiometric ratio indicating the number of $N_2O$ molecules adsorbed to one Cu metal atom in accordance with the following expression (4):

$$D \ (\%) = (K/S) \times (1/R) \times 100 \qquad (4)$$

<CuO crystallite size>

Each of the complex oxides was analyzed by X-ray diffraction (XRD) with a commercial powder X-ray diffractor (SmartLab, which was available from Rigaku Corporation) to obtain the X-ray diffraction pattern of each of the complex oxides. The XRD was performed under the following conditions:

X-ray source: $CuK\alpha$,
Operating axis: $2\theta/\theta$,
Measuring method: Continuous,
Counting unit: cps,
Start angle: 5°,
End angle: 80°,
Sampling width: 0.02°,
Scanning speed: 10°/minute,
Voltage: 40 kV, and
Current: 150 mA.

In the X-ray diffraction pattern of each of the complex oxides, a peak was identified that was present at $2\theta = 38.8°$ $\pm 0.5°$ among the diffraction peaks derived from CuO. The Scherrer equation was applied to the identified peak to calculate the crystallite size. The crystallite size found from the peak present at $2\theta = 38.8°$ $\pm 0.5°$ was defined as the CuO crystallite size. Note that the Scherrer equation is as follows:

$$D = K \times \lambda/B\cos\theta$$

wherein "D" represents the crystallite size (nm), "K" represents the Scherrer constant, "$\lambda$" represents the wavelength of the X-ray (nm), "B" represents the broadening of the diffraction line width (rad), and "$\theta$" represents the Bragg angle (rad).

[Test Example 2]

Each of the complex oxides obtained in Examples 1 to 3 and Comparative Examples 1 to 4 was measured for $CO_2$ methanation catalytic performance ($CO_2$ conversion rate and methanol yield). The measuring method was as follows.
In order to reduce an oxide of Cu (namely CuO) contained in each of the complex oxides to thereby form Cu having a valence lower than before the reduction, each of the complex oxides was heat-treated in a 100% $H_2$ atmosphere at 100°C for 1 hour, and then heat-treated in a 100% $H_2$ atmosphere at 300°C for 1 hour. The catalytic activity of each of the heat-treated complex oxides was evaluated using a reaction tube made of SUS316 and having an inner diameter of 10.22 mm under the following conditions:

Sample volume: 2 $cm^3$,
Sample size: a mesh size of 500 $\mu$m to 1 mm,
Total flow rate of evaluation gas: 100 L/hr ($CO_2$: 25 L/hr, $H_2$: 75 L/hr, $CO_2$:$H_2$ = 1:3),
Space velocity (SV): 50,000 (1/h),
Temperature: 250°C, and
Pressure: 15 MPa.

On-line gas from the outlet of the reaction tube was quantified with a GC-FID (gas chromatography-flame ionization detector) to find the $CO_2$ conversion rate and the methanol yield. The $CO_2$ conversion rate was found from the amount of $CO_2$ reduced. The methanol yield was found from the amount of methanol quantified. The results are shown in Table 1.

[Table 1]

| | Metal elements in complex oxide | | | | |
|---|---|---|---|---|---|
| | Cu (mol%) | Zn (mol%) | Zr (mol%) | Mg (mol%) | Al (mol%) |
| Example 1 | 65 | 24 | 11 | 0 | 0 |
| Example 2 | 68 | 25 | 4.6 | 2.0 | 0 |
| Example 3 | 57 | 22 | 9.7 | 0 | 11 |
| Comparative Example 1 | 64 | 23 | 14 | 0 | 0 |
| Comparative Example 2 | 59 | 21 | 18 | 2.1 | 0 |
| Comparative Example 3 | 60 | 22 | 7.3 | 0 | 10 |
| Comparative Example 4 | 70 | 25 | 5.0 | 0 | 0 |

[Table 1 (continued)]

| | Physical properties of complex oxide | | |
|---|---|---|---|
| | BET specific surface area ($m^2$/g) | Cu dispersity (%) | Crystallite size (nm) |
| Example 1 | 104 | 7.8 | 4.7 |
| Example 2 | 114 | 10.6 | 4.9 |
| Example 3 | 115 | 6.6 | 6.4 |
| Comparative Example 1 | 70 | 5.8 | 5.1 |
| Comparative Example 2 | 85 | 5.2 | 7.5 |
| Comparative Example 3 | 121 | 6.1 | 6.5 |
| Comparative Example 4 | 59 | 4.9 | 11.2 |

[Table 1 (continued)]

| | Catalytic activity of complex oxide | |
|---|---|---|
| | $CO_2$ conversion rate (%) | Methanol yield (%) |
| Example 1 | 29.0 | 20.0 |
| Example 2 | 35.1 | 25.3 |
| Example 3 | 27.6 | 22.2 |
| Comparative Example 1 | 23.0 | 16.0 |
| Comparative Example 2 | 26.1 | 19.2 |
| Comparative Example 3 | 23.0 | 15.6 |
| Comparative Example 4 | (Unmeasured) | (Unmeasured) |

As shown in Table 1, the complex oxides obtained in Examples 1 to 3 have both physical property 1: a specific surface area of 50 $m^2$/g or more and physical property 2: a Cu dispersity of 6.5% or more. The complex oxides obtained in Comparative Examples 1 to 4 do not, however, have at least one of the physical properties 1 and 2.

As shown in Table 1, the complex oxides obtained in Examples 1 to 3 have higher $CO_2$ methanation catalytic performance ($CO_2$ conversion rate and methanol yield) than the complex oxides obtained in Comparative Examples 1 to 4.

**Claims**

1. A complex oxide containing Cu, Zn and Zr, wherein the complex oxide has a specific surface area of 50 $m^2$/g or more and a Cu dispersity of 6.5% or more.

2. The complex oxide as claimed in claim 1, wherein Cu, Zn and Zr contents in the complex oxide are 40% by mole or more and 90% by mole or less, 5% by mole or more and 40% by mole or less, and 1% by mole or more and 15% by mole or less, respectively, based on a total content of all metal elements in the complex oxide.

3. The complex oxide as claimed in claim 1 or 2, further containing one or two metal elements selected from Mg and Al.

4. The complex oxide as claimed in claim 1 or 2, wherein the complex oxide has a CuO crystallite size of 2.0 nm or more and 25.0 nm or less.

5. A catalyst for methanol production, comprising the complex oxide as claimed in claim 1 or 2.

6. A method for producing a complex oxide, the method comprising the following steps of:

   (a) adding an acidic aqueous solution containing two or more metal ions to an alkaline aqueous solution having a pH of 7.5 or more and 11 or less to form a precipitate containing two or more metal elements; and
   (b) calcining the precipitate to produce a complex oxide containing the two or more metal elements.

7. The production method as claimed in claim 6, wherein, in step (a), the alkaline aqueous solution after the addition of the acidic aqueous solution has a pH of 5 or more and 9 or less.

8. The production method as claimed in claim 6 or 7, wherein the acidic aqueous solution used in step (a) contains Cu ion, Zn ion and Zr ion, and the complex oxide produced in step (b) contains Cu, Zn and Zr.

9. The production method as claimed in claim 8, wherein the complex oxide produced in step (b) further contains one or two metal elements selected from Mg and Al.

10. The production method as claimed in claim 8, wherein the complex oxide produced in step (b) is the complex oxide as claimed in claim 1 or 2.

11. A method for producing methanol, the method comprising a step of contacting $CO_2$ gas with $H_2$ gas under conditions of 100°C or more and 400°C or less and 0.1 MPa or more and 30 MPa or less in the presence of the complex oxide as claimed in claim 1 or 2 subjected to a reduction treatment.

## INTERNATIONAL SEARCH REPORT

| International application No. |
| --- |
| **PCT/JP2023/028367** |

| A. | CLASSIFICATION OF SUBJECT MATTER |
| --- | --- |

***B01J 23/80*** (2006.01)i; ***B01J 35/61*** (2024.01)i; ***B01J 37/03*** (2006.01)i; ***B01J 37/08*** (2006.01)i; ***B01J 37/18*** (2006.01)i; ***C01G 25/00*** (2006.01)i; ***C07B 61/00*** (2006.01)i; ***C07C 29/153*** (2006.01)i; ***C07C 31/04*** (2006.01)i

FI: B01J23/80 Z; B01J35/10 301J; B01J37/03 B ZAB; B01J37/08; B01J37/18; C01G25/00; C07B61/00 300; C07C29/153; C07C31/04

According to International Patent Classification (IPC) or to both national classification and IPC

| B. | FIELDS SEARCHED |
| --- | --- |

Minimum documentation searched (classification system followed by classification symbols)

B01J23/80; B01J35/10; B01J37/03; B01J37/08; B01J37/18; C01G25/00; C07B61/00; C07C29/153; C07C31/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580/JSTChina (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 102259002 A (CHINA PETROLEUM AND CHEMICAL CORP.) 30 November 2011 (2011-11-30) claims, paragraphs [0006], [0023]-[0041], [0047]-[0053], tables 2, 3 | 1-2, 4-8, 10-11 |
| Y | claims, paragraphs [0006], [0023]-[0041], [0047]-[0053], tables 2, 3 | 3, 9 |
| X | JP 2001-347169 A (NATIONAL INSTITUTE OF ADVANCED INDUSTRIAL SCIENCE AND TECHNOLOGY) 18 December 2001 (2001-12-18) claims, table 1 | 1, 3 |
| Y | JP 9-141099 A (MITSUBISHI HEAVY INDUSTRIES, LTD.) 03 June 1997 (1997-06-03) claims, paragraphs [0008]-[0024] | 3, 9 |
| Y | JP 10-216522 A (KANSAI ELECTRIC POWER CO., INC.) 18 August 1998 (1998-08-18) claims | 3, 9 |
| A | WO 2013/183577 A1 (MITSUI CHEMICALS, INC.) 12 December 2013 (2013-12-12) claims, paragraphs [0107], [0119]-[0123] | 1-11 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 October 2023** | **24 October 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2023/028367**

### C. DOCUMENTS CONSIDERED TO BE RELEVANT

| Category* | Citation of document, with indication, where appropriate, of the relevant passages | Relevant to claim No. |
|---|---|---|
| A | JP 2015-513454 A (BASF CORP.) 14 May 2015 (2015-05-14)<br>entire text | 1-11 |
| A | JP 2011-528617 A (HYUNDAI HEAVY INDUSTRIES CO., LTD.) 24 November 2011 (2011-11-24)<br>entire text | 1-11 |

Form PCT/ISA/210 (second sheet) (January 2015)

**INTERNATIONAL SEARCH REPORT**
Information on patent family members

International application No.

**PCT/JP2023/028367**

| Patent document cited in search report | | | Publication date (day/month/year) | Patent family member(s) | | | Publication date (day/month/year) |
|---|---|---|---|---|---|---|---|
| CN | 102259002 | A | 30 November 2011 | (Family: none) | | | |
| JP | 2001-347169 | A | 18 December 2001 | US | 2002/0051747 | A1 | |
| | | | | claims, table 1 | | | |
| | | | | EP | 1161992 | A1 | |
| JP | 9-141099 | A | 03 June 1997 | (Family: none) | | | |
| JP | 10-216522 | A | 18 August 1998 | (Family: none) | | | |
| WO | 2013/183577 | A1 | 12 December 2013 | US | 2015/0111975 | A1 | |
| | | | | claims, paragraphs [0107], [0119]-[0123] | | | |
| | | | | EP | 2857095 | A1 | |
| | | | | CN | 104379255 | A | |
| JP | 2015-513454 | A | 14 May 2015 | US | 2013/0211148 | A1 | |
| | | | | entire text | | | |
| | | | | EP | 2814889 | A1 | |
| | | | | KR | 10-2014-0133849 | A | |
| JP | 2011-528617 | A | 24 November 2011 | US | 2011/0118367 | A1 | |
| | | | | entire text | | | |
| | | | | EP | 2305379 | A2 | |
| | | | | CN | 102105222 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7039755 A